# EUROPEAN PATENT SPECIFICATION

(11) **EP 1 585 756 B1**
(45) Date of publication and mention of the grant of the patent: **21.04.2010**
(21) Application number: 03796481.4
(22) Date of filing: 24.11.2003
(51) Int. Cl.: C12N 15/11

(54) **DELIVERY OF SIRNAS**
VERABREICHUNG VON SIRNAS
ADMINISTRATION DE SIRNAS

(30) Priority: 26.11.2002 US 430520 P
(43) Date of publication of application: 19.10.2005
(73) Proprietor: University of Massachusetts, Boston, MA 02108 (US)
(72) Inventor: RANA, Tariq, M., Shrewsbury, MA 01545 (US)
(74) Representative: Kirkham, Nicholas Andrew
(86) International application number: PCT/US2003/037886
(87) International publication number: WO 2004/048545

(56) References cited:
- WO-A-02/10201
- WO-A-02/20060
- WO-A1-95/02397
- WO-A2-02/44321
- WO-A2-03/064625
- US-A1- 2002 137 064
- US-B2- 6 632 616
- SCHWARTZ J ET AL: "PEPTIDE-MEDIATED CELLULAR DELIVERY" CURRENT OPINION IN MOLECULAR THERAPEUTICS, CURRENT DRUGS, LONDON,, GB, vol. 2, no. 2, April 2000 (2000-04), pages 162-167, XP001037249 ISSN: 1464-8431
- ASTRIAB-FISHER A ET AL: "Conjugates of antisense oligonucleotides with the Tat and Antennapedia cell-penetrating peptides: Effects on cellular uptake, binding to target sequences, and biologic actions" PHARMACEUTICAL RESEARCH, NEW YORK, NY, US, vol. 19, no. 6, June 2002 (2002-06), pages 744-754, XP002259727 ISSN: 0724-8741
- YOO H ET AL: "PAMAM DENDRIMERS AS DELIVERY AGENTS FOR ANTISENSE OLIGONUCLEOTIDES" PHARMACEUTICAL RESEARCH, NEW YORK, NY, US, vol. 16, no. 12, 1999, pages 1799-1804, XP001030542 ISSN: 0724-8741
- BERTRAND J R ET AL: "Comparison of antisense oligonucleotides and siRNAs in cell culture and in vivo" BIOCHEMICAL AND BIOPHYSICAL RESEARCH COMMUNICATIONS, ACADEMIC PRESS, SAN DIEGO, CA, US, vol. 296, no. 4, 30 August 2002 (2002-08-30), pages 1000-1004, XP002994742 ISSN: 0006-291X
- SIMEONI, F. ET AL.: 'Insight into the mechanism of the peptide-based gene delivery system MPG: implications for delivery of siRNA into mammalian cells' NUCLEIC ACID RESEARCH vol. 31, 13 March 2003, pages 2717 - 2724, XP002984580
- BIELINKSKA, A. ET AL.: 'Regulation of in vitro gene expression using antisense oligonucleotides or antisense expression plasmids transfected using starburst PAMAM dendrimers' NUCLEIC ACIDS RESEARCH vol. 24, 30 November 1995, pages 2176 - 2182, XP000589893
- NORMAND, N. ET AL.: 'Particle formation by a conserved domain of the herpes simplex virus protein vp22 facilitating protein and nucleic acid delivery' JBC vol. 276, 13 November 2000, pages 15042 - 15050, XP002259726
- MORRIS, M.C. ET AL.: 'A novel potent strategy for gene delivery using a single peptide vector as a carrier' NUCLEIC ACIDS RESEARCH vol. 27, 07 May 1999, pages 3510 - 3517, XP002240390
- GARCIA-CHAUMONT, C. ET AL.: 'Delivery systems for antisense oligonucleotides' PHARMACOLOGY & THERAPEUTICS vol. 87, December 2000, XP002984581
- LINDSAY, M.A.: 'Peptide-mediated cell delivery: application in protein target validation' CURRENT OPINION IN PHARMACOLOGY vol. 2, October 2002, pages 587 - 594, XP008041390
- ELBASHIR, ET AL.: 'RNA interference is mediated by 21- and 22- nucleotide RNAs' GENES AND DEVELOPMENT vol. 15, 25 October 2000, pages 188 - 200, XP002204651
- TOMALIA D.A.: 'Birth of a New Macromolecular Architecture: Dendrimers as Quantized Building Blocks for Nanoscale Synthetic Organic Chemistry' ALDRICHIMICAACTA vol. 37, no. 2, 2004, pages 39 - 57
- EICHMAN J.D. ET AL: 'The use of PAMAM dendrimers in the efficient transfer of genetic material into cells' PHARMACEUTICAL SCIENCE AND TECHNOLOGY TODAY vol. 3, July 2000, pages 232 - 244
- HAENSLER J.; SZOKA F.C.: 'POLYAMIDOAMINE CASCADE POLYMERS MEDIATE EFFICIENT TRANSFECTION TO CELLS IN CULTURE' BIOCONJUGATE CHEMISTRY vol. 4, 01 January 1993, ACS, WASHINGTON, DC, US, pages 372 - 379, XP002905764
- TANG M.X. ET AL: 'In vitro gene delivery by degraded polyamidoamine dendrimers.' BIOCONJUGATE CHEMISTRY vol. 7, 01 November 1996, ACS, WASHINGTON, DC, US, pages 703 - 714, XP000638685
- SVENSON S.; TOMALIA D.A.: 'Dendrimers in biomedical applications-reflections on the field' ADVANCED DRUG DELIVERY REVIEWS vol. 57, 14 December 2005, ELSEVIER BV, AMSTERDAM, NL, pages 2106 - 2129, XP005202708
- STEVENS M.P.: 'Polymer Chemistry: An Introduction', 1998, OXFORD UNIVERSITY PRESS, NEW YORK, NY, US * page 301 - page 307 *

## Description

### TECHNICAL FIELD

This invention relates to delivery of siRNAs.

### BACKGROUND

RNA interference (RNAi) is a powerful and specific method for silencing or reducing the expression of a target gene, mediated by small single- or double-stranded RNA molecules. These molecules include small interfering RNAs (siRNAs), microRNAs (miRNAs), small hairpin RNAs (shRNAs), and others. Although the mechanism by which RNAi functions is not fully elucidated, it is clear that RNAi is a promising method of treatment, e.g., by targeting specific mRNAs for elimination. One obstacle to the development of RNAi technology for therapeutic uses has been that most methods of delivering RNAs that mediate RNAi are toxic to cells *in vitro* and *in vivo*.

### SUMMARY

The present invention is based, in part, upon the discovery of siRNA delivery methods using delivery peptides or chemical agents with little or no toxicity, e.g., suitable for use in vivo. The invention is defined in the claims.

There is described, a method for delivering an siRNA or engineered RNA precursor to a cell by obtaining a cell, conjugating at least one delivery peptide to an siRNA or engineered RNA precursor to form a peptide-conjugate, and contacting the cell with the peptide-conjugate. The delivery peptide may be a Tat peptide. The delivery peptide may have a sequence substantially similar to the sequence of SEQ ID NO. 12. The delivery peptide can be homeobox (hox) peptide, an MTS, VP22, and/or MPG.

There is also disclosed, a method for delivering an siRNA to a cell by obtaining a cell, forming a mixture comprising an siRNA and at least one dendrimer and contacting the cell with the mixture. In one embodiment, the dendrimer is PAMAM.

A kit for conjugating a delivery peptide to an siRNA, comprising the delivery peptide and an activating agent is also disclosed. In one alternative, the kit contains a Tat, homeobox (hox), MTS, MPG, and/or VP22 delivery peptide.

A kit for preparing an siRNA delivery mixture comprising a dendrimer and instructions for use in mixing with an siRNA is also disclosed, and the dendrimer may be PAMAM.

An siRNA delivery mixture comprising a dendrimer is also disclosed.

An siRNA or engineered RNA precursor conjugated to a delivery peptide is disclosed and the delivery peptide may be Tat, homeobox (hox), VP22, MPG, and/or MST.

There is also disclosed, biconjugates of targeting peptides, e.g., homeobox (hox) peptides, TAT peptides, membrane translocating sequence, Penetratin™ and/or transportin, which enhance uptake of siRNA and thus promote gene silencing *in vivo.* These peptides are suitable for use in living cells.

In another aspect, the invention features dendrimers, e.g., polyamidoamines (PAMAM) dendrimers, which enhance uptake of siRNA and are suitable for promoting gene silencing *in vivo*.

A "target gene" is a gene whose expression is to be selectively inhibited or "silenced." This silencing is achieved by cleaving the mRNA of the target gene by an siRNA, *e*.*g*., an isolated siRNA or one that is created from an engineered RNA precursor. One portion or segment of a duplex stem of the siRNA RNA precursor, or one strand of the siRNA, is an anti-sense strand that is complementary, e.g., fully complementary, to a section, e.g., about 16 to about 40 or more nucleotides, of the mRNA of the target gene.

An "isolated nucleic acid molecule or sequence" is a nucleic acid molecule or sequence that is not immediately contiguous with both of the coding sequences with which it is immediately contiguous (one on the 5' end and one on the 3' end) in the naturally occurring genome of the organism from which it is derived. The term therefore includes, for example, a recombinant DNA or RNA that is incorporated into a vector; into an autonomously replicating plasmid or virus; or into the genomic DNA of a prokaryote or eukaryote, or which exists as a separate molecule (e.g., a cDNA or a genomic DNA fragment produced by PCR or restriction endonuclease treatment) independent of other sequences. It also includes a recombinant DNA that is part of a hybrid gene encoding an additional polypeptide sequence.

The term "engineered," as in an engineered RNA precursor, or an engineered nucleic acid molecule, indicates that the precursor or molecule is not found in nature, in that all or a portion of the nucleic acid sequence of the precursor or molecule is created or selected by man. Once created or selected, the sequence can be replicated, translated, transcribed, or otherwise processed by mechanisms within a cell. Thus, an RNA precursor produced within a cell from an engineered nucleic acid molecule, e.g., a transgene, is an engineered RNA precursor. Engineered RNA precursors are artificial constructs that are similar to naturally occurring precursors of small temporal RNAs (stRNAs) that are processed in the body to form siRNAs. The engineered RNA precursors can be synthesized by standard methods known in the art, e.g. by use of an automated DNA synthesizer (such as are commercially available from Biosearch, Applied Biosystems, etc.) or encoded by nucleic acid molecules.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

FIG. 1A is a line graph of Cy3 fluorescence intensity of nucleic acids isolated from cells transfected with Cy3-labeled CDK9 siRNA, either by PAMAM or Lipofectamine™.
FIG. 1B is a bar graph of the peak fluorescence intensity at 570 nM for each of the conditions shown in figure 1A.
FIG. 2 is a phosphorimage of an immunoblot of human Cyclin T1 (hCycT1) and cyclin-dependent kinase 9 (CDK9) expression in cells transfected with Cy3-labeled CDK9 siRNA, either by PAMAM.
FIG. 3A is a line graph of Cy3 fluorescence intensity of nucleic acids isolated from cells transfected with TAT-modified Cy3-labeled CDK9 siRNA or control unmodified Cy3-labeled CDK9 siRNA, transfected using Lipofectamine™.
FIG. 3B is a bar graph of the peak fluorescence intensity at 570 nM for each of the conditions shown in figure 3A.
FIG. 4 is a bar graph of the ratio of fluorescence intensity of target enhanced Green Fluorescent Protein (EGFP) to control Red Fluorescent Protein (RFP) fluorophore.
FIG. 5 is a phosphorimage of an immunoblot of human Cyclin T1 (hCycT1) and cyclin-depeodent kinase 9 (CDK9) expression in cells transfected with TAT-modified Cy3-labeled CDK9 siRNA or control unmodified Cy3-labeled CDK9 siRNA transfected using Lipofectamine™.
FIG. 6A is a drawing of the structure of a highly branched dendrimer.
FIG. 6B is a drawing of the structure of a less branched dendrimer.
FIG. 6C is a drawing of the structure of a PEG dendrimer.
FIG. 7 depicts the sequence [SEQ ID NO.12: CYGRKKRRQRRR] and structure of a Tat delivery peptide.
FIG 8A is a fluorescent image of HeLa cells transfected using Lipofectamine™ with Cy3-SS/AS Duplex siRNA.
FIG 8B is a Nomarski Differential Interference (DIC) of the same HeLa cells shown in 8A transfected using Lipofectamine™ with Cy3-SS/AS Duplex siRNA.
FIG 8C is a pseudocolored overlay of the fluorescent image of FIG 8A and the Nomarksi Differential Interference (DIC) of FIG 8C.
FIG 8D is a fluorescent image of HeLa cells transfected using Lipofectamine™ with Cy-3-SS/AS Duplex siRNA.
FIG 8E is a Nomarski Differential Interference (DIC) of the same HeLa cells shown in 8D transfected using Lipofectamine™ with Cy3-SS/AS Duplex siRNA.
FIG 8F is a pseudocolored overlay of the fluorescent image of FIG 8D an the Nomarski Differential Interference (DIC) of FIG 8E.
FIG 9A is a fluorescent image of HeLa cells transfected with Cy3-SS/AS Duplex siRNA using dendrimer (PAMAM)-mediated delivery.
FIG 9B is a Nomarski Differential Interference (DIC) of the same HeLa cells shown in 9A transfected with Cy3-SS/AS Duplex siRNA using dendrimer (PAMAM)-mediated delivery.
FIG 9C is a psuedocolored overlay of the fluorescent image of FIG. 9A and the Nomarksi Differential Interference (DIC) of FIG 9C.
FIG 9D is a fluorescent image of HeLa cells transfected with Cy3-SS/AS Duplex siRNA using dendrimer (PAMAM)-mediated delivery.
FIG 9E is a Nomarski Differential Interference (DIC) of the same HeLa cells shown in 9D transfected with Cy3-SS/AS Duplex siRNA using dendrimer (PAMAM)-mediated delivery.
FIG 9F is a pseudocolored overlay of the fluorescent image of FIG 9D and the Nomarski Differential Interference (DIC) of Fig. 9E.
FIG 9G is a fluorescent image of HeLa cells transfected with Cy3-SS/AS Duplex siRNA using dendrimer (PAMAM)-mediated delivery.
FIG 9H is a Nomarski Differential Interference (DIC) of the same HeLa cells shown in 9G transfected with Cy3-SS/AS Duplex siRNA using dendrimer (PAMAM)-mediated delivery.
FIG 9I is a pseudocolored overlay of the fluorescent image of FIG 9G and the Nomarski Differential Interference (DIC) of FIG 9H.
FIG 10A is a fluorescent image ofHeLa cells transfected with Cy3-SS/AS-TAT (47-57) Duplex siRNA.
FIG 10B is a Nomarski Differential Interference (DIC) of the same HeLa cells shown in 10A transfected with Cy3-SS/AS-TAT (47-57) Duplex siRNA.
FIG. 10C is a pseudocolored overlay of the fluorescent image of FIG. 10A and the Nomarski Differential Interference (DIC) of FIG 10C.
FIG 10D is a fluorescent image of HeLa cells transfected with Cy3-SS/AS-TAT (47-57) Duplex siRNA.
FIG 10E is a Nomarski Differential Interference (DIC) of the same HeLa cells shown in 10D transfected with Cy3-SS/AS-TAT (47-57) Duplex siRNA.
FIG 10F is a pseudocolored overlay of the fluorescent image of FIG. 10D and the Nomarski Differential Interference (DIC) of FIG. 10E.
FIG 10G is a fluorescent image of HeLa cells transfected with Cy3-SS/AS-TAT (47-57) Duplex siRNA.
FIG 10H is a Nomarski Differential Interference (DIC) of the same HeLa cells shown in 10G transfected with Cy3-SS/AS-TAT (47-57) Duplex siRNA.
FIG 10I is a pseudocolored overlay of the fluorescent image of FIG. 10G and the Nomarski Differential Interference (DIC) of FIG. 10H.
FIG 11 depicts the sequence of the sense strand [SEQ ID NO. 13] and antisense strand [SEQ ID NO. 14] of the EGFP duplex siRNA

### DETAILED DESCRIPTION

The present invention provides compositions for delivering siRNAs, or siRNA precursors, into cells, e.g., eukaryotic cells such as mammalian cells (for example, human cells).

Sequence-selective, post-transcriptional inactivation of expression of a target gene can be achieved in a wide variety of eukaryotes by introducing double-stranded RNA corresponding to the target gene, a phenomenon termed RNA interference (RNAi). This approach takes advantage of the discovery that siRNA can trigger the degradation ofmRNA corresponding to the siRNA sequence. To be effective, the siRNA must not only enter the cell, but must also enter the cell in sufficient quantities to have a significant effect. RNAi methodology has been extended to cultured mammalian cells, but its application *in vivo* has been limited due to a lack of efficient delivery systems with little or not toxicity. The present application provides such a system.

At present most commonly used techniques (such as microinjection, transfection using cationic liposomes, viral transfection or electroporation of oligonucleotide conjugates) induce in the cells and/or host stress and other limitations and drawbacks. For example, nucleic acid delivery mediated by cationic liposomes such as LIPOFECTAMINE™, LIPOFECTIN™, CYTOFECTIN™ as well as transfection mediated by polymeric DNA-binding cations such as poly-L-lysine or polyethyleneimine are extensively used transfection techniques. These methods can be associated with cytotoxicity and sensitivity to serum, antibiotics and certain cell culture media. In addition, these methods are limited by low overall transfection efficiency and time-dependency Other methods such as microinjection or electroporation are simply not suitable for large-scale delivery of nucleic acids into living tissues.

### RNA Interference

RNAi is a remarkably efficient process whereby double-stranded RNA (dsRNA) induces the sequence-specific degradation of homologous mRNA in animals and plant cells (Hutvagner and Zamore (2002), Curr. Opin. Genet. Dev., 12, 225-232; Sharp (2001), Genes Dev., 15, 485-490). In mammalian cells, RNAi can be triggered by 21-nucleotide (nt) duplexes of small interfering RNA (siRNA) (Chiu et al. (2002), Mol. Cell., 10, 549-561; Elbashir et al. (2001), Nature, 411, 494-498), or by micro-RNAs (miRNA), functional small-hairpin RNA (shRNA), or other dsRNAs that are expressed *in vivo* using engineered RNA precursors such as DNA templates, e.g., with RNA polymerase III promoters (Zeng et al. (2002), Mol. Cell, 9, 1327-1333; Paddison et al. (2002), Genes Dev., 16, 948-958; Lee et al. (2002), Nature Biotechnol., 20, 500-505; Paul et al. (2002), Nature Biotechnol., 20, 505-508; Tuschl, T. (2002), Nature Biotechnol., 20,440-448; Yu et al. (2002), Proc. Natl. Acad. Sci. USA, 99(9), 6047-6052; McManus et al. (2002), RNA, 8, 842-850; Sui et al. (2002), Proc. Natl. Acad. Sci. USA, 99(6), 5515-5520.)

### siRNA Molecules

The nucleic acid molecules or constructs useful in the invention include dsRNA molecules comprising 16-30, e.g., 16, 17, 18, 19, 20, 21, 22, 23, 24, 25, 26, 27, 28, 29, or 30 nucleotides in each strand, wherein one of the strands is substantially complementary to, e.g., at least 80% (or more, e.g., 85%, 90%, 95%, or 100%) (for example, having 3, 2, 1, or 0 mismatched nucleotide(s)), to a target region, such as a target region that differs by at least one base pair between the wild type and mutant allele of a nucleic acid sequence. For example, the target region can comprise a gain-of-function mutation, and the other strand is identical or substantially identical to the first strand. The dsRNA molecules useful in the invention can be chemically synthesized, or can be transcribed *in vitro* from a DNA template, or *in vivo* from an engineered RNA precursor, e.g., shRNA. The dsRNA molecules can be designed using any method known in the art, for instance, by using the following protocol:
1. Beginning with the AUG start codon of, look for AA dinucleotide sequences; each AA and the 3' adjacent 16 or more nucleotides are potential siRNA targets. The siRNA should be specific for a target region that differs by at least one base pair between the wild type and mutant allele, e.g., a target region comprising the gain of function mutation. The first strand should be complementary to this sequence, and the other strand is identical or substantially identical to the first strand. In one embodiment, the nucleic acid molecules are selected from a region of the target allele sequence beginning at least 50 to 100 nt downstream of the start codon, e.g., of the sequence of SOD1. Further, siRNAs with lower G/C content (35-55%) may be more active than those with G/C content higher than 55%. Thus in one embodiment, the invention includes nucleic acid molecules having 35-55% G/C content. In addition, the strands of the siRNA can be paired in such a way as to have a 3' overhang of 1 to 4, e.g., 2, nucleotides. Thus in another embodiment, the nucleic acid molecules may have a 3' overhang of 2 nucleotides, such as TT. The overhanging nucleotides may be either RNA or DNA. In one embodiment, the overhang nucleotides are deoxythymidines or other appropriate nucleotides or nucleotide analogs. Other embodiments are also envisioned where the strands of the siRNA do not have a 3' overhang. As noted above, it is desirable to choose a target region wherein the mutant:wild type mismatch is a purine:purine mismatch.
2. Using any method known in the art, compare the potential targets to the appropriate genome database (human, mouse, rat, etc.) and eliminate from consideration any target sequences with significant homology to other coding sequences. One such method for such sequence homology searches is known as BLAST, which is available at www.ncbi.nlm.nih.gov/BLAST.
3. Select one or more sequences that meet your criteria for evaluation. Further general information about the design and use of siRNA may be found in "The siRNA User Guide," available at www.mpibpc.gwdg.de/abteiluagen/100/105/sirna.html.

Negative control siRNAs should have the same nucleotide composition as the selected siRNA, but without significant sequence complementarity to the appropriate genome. Such negative controls may be designed by randomly scrumbling the nucleotide sequence of the selected siRNA; a homology search can be performed to ensure that the negative control lacks homology to any other gene in the appropriate genome. In addition, negative control siRNAs can be designed by introducing one or more base mismatches into the sequence.

The siRNAs useful in the invention include both siRNA and crosslinked siRNA derivatives as described in WO2004/29212. Crosslinking can be employed to alter the pharmacokinetics of the composition, for example, to increase balf-life in the body. Thus, the invention includes siRNA derivatives that include siRNA having two complementary strands of nucleci acid, such that the two strands are crosslinked. For example, a 3' OH terminus of one of the strands can be modified, or the two strands can be crosslinked and modified at the 3' OH terminus. The siRNA derivative can contain a single crosslink (e.g., a psoralen crosslink). In some embodiments, die siRNA derivates has at its 3' terminus a biotin molecule (e.g., a photocleavable biotin), a peptide (e.g., a Tat peptide), a nonoparticle, a peptidomimetic, organic compounds (e.g., a dye such as a fluorescent dye), or dendrimer. Modifying siRNA derivatives in this way may improve cellular uptake or enhance cellular targeting activities of the resulting siRNA derivative as compared to the corresponding siRNA, are useful for tracing the siRNA derivative in the cell, or improve the stability of the siRNA derivative compared to the corresponding siRNA.

The nucleic acid molecules useful in the present invention can also be labeled using any method known in the art; for instance, the nucleic acid compositions can be labeled with a fluorophore, e.g., Cy3, fluorescein, or rhodamine. The labeling can be carried out using a kit, e.g., the SILENCER™ siRNA labeling kit (Ambion). Additionally, the siRNA can be radiolabeled, e.g., using ³H, ³²P, or other appropriate isotope.

Nucleic acid molecules recited herein comprise nucleotide sequences as set forth in the sequence listing with or without 3' overhangs, *e*.*g*., with or without 3'-deoxythymidines. Other embodiments are also envisioned in which the 3' overhangs comprise other nucleotides, e.g., UU or the like.

### SiRNA-Delivery of Peptide Conjugates

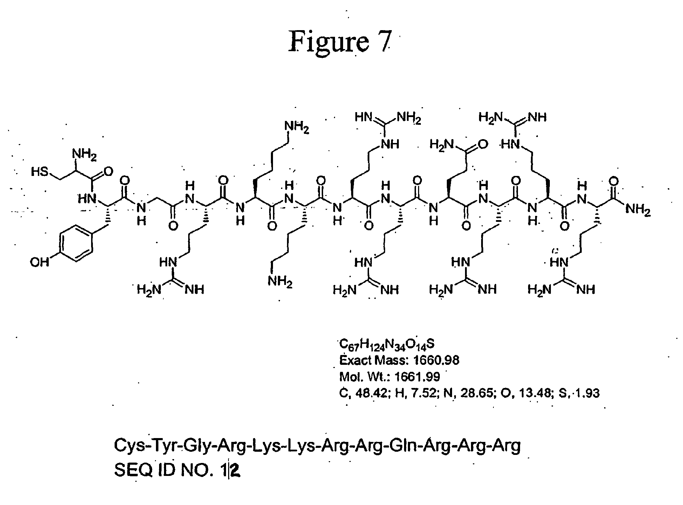

The siRNAs described herein, as well as an engineered RNA precursor or engineered nucleic acid molecules that encode the precursors, can be conjugated to delivery peptides or other compounds to enhance the efficiency of transport of the siRNA into living cells compared to the efficiency of delivery to unmodified siRNA. These delivery peptides can include peptides known in the art to have cell-penetrating properties. For instance, the delivery peptide can be, but is not limited to: TAT derived short peptide from human immunodeficiency virus (HIV-1), such as TAT 47-57 and Cys [SEQ ID NO. 12: CYGRKKRRQRRR] (see also Figure 7), and TAT 49-60 and (Arg)9 (Tat) [SEQ ID NO. 1: RKKRRQRRRPPQC] (Reference 4-7, 23), and substantially similar variants thereof, e.g., a variant that is at least 65% identical thereto. Of course, the percent identity can be higher, *e*.*g*., 65%, 67%, 69%, 70%, 73%, 75%, 77%, 83%, 85%, 87%, 90%, 93%, 95%, 97%; 100% identity (for example, peptides with substitutions at 1, 2, 3, 4 or more residues) (*e*.*g*., SEQ ID NO: 16: CYQRKKRRQRRR). In general, the substitutions are conservative substitutions. The methods of making such peptides are routine in the art.

The delivery peptide can also be, but is not limited to: the third α-helix of Drosophila Antennapedia homeodomain (Ant) [SEQ ID NO. 2: RQIKIWFQNRRMKWKKGGC] and substantially similar variants thereof (Reference 8, 18, 21); VP22 protein from herpes simplex virus [SEQ ID NO. 3: DAATATRGRSAASRPTERPRAPARSASRPRRPVE] and substantially similar variants thereof (Reference 9); Nuclear localization sequence (NLS) of simian virus 40 (SV-40) large T antigen and substantially similar variants thereof (Reference 10); designed peptides (synthetic and/or chimeric cell-penetrating peptides) and variants thereof, including the Pep-1 peptide, a 21-residue peptide carrier [SEQ ID NO. 4: KETWWETWWTEWSQ-PKKKRKV] consisting of three domains: (1) a hydrophobic tryptophan-rich motif, for efficient targeting to the cell membrane; (2) NLS of SV40 large T antigen, to improve intracellular delivery and solubility of the peptide vector; and (3) a spacer domain (SQP), containing a proline residue, to improve the flexibility and the integrity of the two hydrophobic and hydrophilic domains mentioned above, and substantially similar variants thereof (Reference 13); the MPG/MPS delivery system a 27 residue synthetic peptide containing a hydrophobic domain derived from the fusion sequence of HIV gp41 and a hydrophilic domain derived from the nuclear localization sequence of SV40 T-antigen [SEQ ID NO. 5: GALFLGWLGAAGSTMGAWSQPKKKRKV] and substantially similar variants thereof (Reference 1,10); membrane translocating sequences (MTSs) derived from the hydrophobic regions of the signal sequences from Kaposi's sarcoma fibroblast growth factor 1 (K-FGF) 18 and human b3 integrin19, the fusion sequence of HIV-1 gp41; the signal sequence of the variable immunoglobulin light chain Ig(v) from Caiman crocodylus21 conjugated to NLS peptides originating from nuclear transcription factor kB (NF-kB)22, Simian virus 40 (SV40) T-antigen23 or K-FGF; cell-penetrating peptide, containing 16 residues from the K-FGF MTS coupled to a F-kB NLS (ten residues) or coupled to the SV40 T-antigen NLS (12 residues), [SEQ ID NO. 6: AAVALLPAV-LLALLAP] and variants thereof; the MTS from Ig(v) light chain coupled via a peptidase sensitive linker to residues 127-132 of SV40 T-antigen and variants thereof; cell-penetrating peptides including but not limited to penetratin, PEN (43-58 of the homeodomain of *D. melanogaster* antennapedia transcription factor, ANTP), SEQ ID NO. 7: RQIKIWFQ-NRRMKWKK] and substantially similar variants thereof (Reference 16); signal-sequence-based peptides (I) [SEQ ID NO. 8: GALFLGWLGAAGSTMGAWSQPKKKRKV] and variants thereof; signal-sequence-based peptides (II) [SEQ ID NO. 9: AAVALLPAVLLALLAP] and variants thereof; transportan [ SEQ ID NO. 10: GWTLNSAGYLLKINLKALAALAKKIL] and variants thereof; galparan, a fusion between the neuropeptide galanin-1-13 and the wasp venom peptide mastoparans and substantially similar variants thereof (Reference 1); amphiphilic model peptide [ SEQ ID NO. 11: KLALKLALKALKAALKLA]; 18-mer amphipathic model peptide27 (Reference 14-15); branched-chain arginine peptides and substantially similar variants thereof (Reference 17); 9-polylysine protein transduction domain and substantially similar variants thereof (Reference 19); b-peptide and variants thereof; shell cross-linked (SCK) nanoparticles combined with the oligomeric peptide sequence of the TAT protein transduction domain and substantially similar variants thereof (Reference 20).

The peptides can also have modified backbones, e.g., oligocarbamate or oligourea backbones; see, e.g., Wang et al., J. Am. Chem. Soc., Volume 119, pp. 6444-6445, (1997); Tamilarasu et al., J. Am. Chem. Soc., Volume 121, pp. 1597-1598, (1999), Tamilarasu et al., Bioorg. Of Med. Chem. Lett., Volume 11, pp. 505-507, (2001).

The conjugation can be accomplished by methods known in the art, e.g., using the methods of Lambert et al. (2001), Drug Deliv. Rev., 47(1), 99-112 (describes nucleic acids loaded to polyalkylcyanoacrylate (PACA) nanoparticles); Fattal et al. (1998), J. Control Release, 53(1-3), 137-43 (describes nucleic acids bound to nanoparticles); Schwab et al. (1994), Ann. Oncol., 5 Suppl. 4, 55-8 (describes nucleic acids linked to intercalating agents, hydrophobic groups, polycations or PACA nanoparticles); and Godard et al. (1995), Eur. J. Biochem., 232(2), 404-10 (describes nucleic acids linked to nanoparticles).

Peptide conjugates recited herein comprise peptide portions as set forth in the sequence listing with or without terminal cysteine residues.

### siRNAs mixed with Delivery Agents

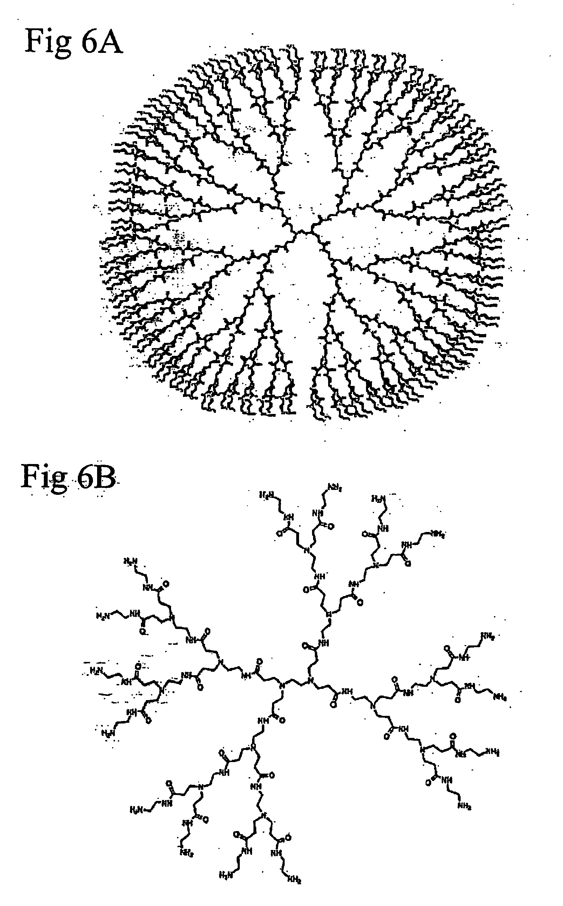

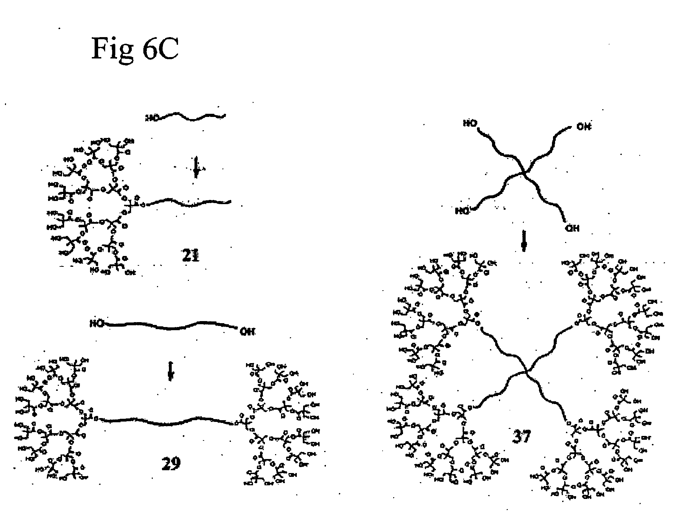

The siRNAs described herein can also be delivered by mixing with a delivery agent, e.g., a dendrimer. Dendrimers are highly branched polymers with well-defined architecture, capable of delivery other compounds into a cell. Three non-limiting examples of dendrimers are shown in FIG. 6A, 6B and 6C. Many dendrimers are commercially available, e.g., from Sigma-Aldrich. The dendrimers useful in the invention include PAMAM: Amine terminated and/or PAMAM: Carboxylic Acid terminated (available, e.g., from Dendritech, Inc., Midland, MI); Diaminobutane (DAB) - DAB: Amine terminated and/or DAB: Carboxylic Acid terminated; PEGs: OH terminated (Figure 6c and Frechet et al. JACS 123:5908 (2001)), among others. In general, PAMAM or a variant thereof is used.

### Pharmaceutical Compositions and Methods of Administration

The siRNA molecules useful in the invention can be incorporated into pharmaceutical compositions. Such compositions typically include the siRNA-peptide conjugate or siRNA and delivery agent mixture, and a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer. Such methods include those described in U.S. Patent No. 6,468,798.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

The compounds can also be administered by any method suitable for administration of nucleic acid agents, e.g., using gene guns, bio injectors, and skin patches as well as needle-free methods such as the micro-particle DNA vaccine technology disclosed in U.S. Patent No. 6,194,389, and the mammalian transdermal needle-free vaccination with powder-form vaccine as disclosed in U.S. Patent No. 6,168,587. Additionally, intranasal delivery is possible, as described in, *inter alia*, Hamajima et al. (1998), Clin. Immunol. Immunopathol., 88(2), 205-10. Liposomes (e.g., as described in U.S. Patent No. 6,472,375) and microencapsulation can also be used. Biodegradable targetable microparticle delivery systems can also be used (e.g., as described in U.S. Patent No. 6,471,996).

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of an siRNA-peptide conjugate or siRNA delivery agent mixture, e.g., an siRNA-dendrimer mixture (i.e., an effective dosage) depends on the nucleic acid selected. For instance, if a plasmid encoding shRNA is selected, single dose amounts in the range of approximately 1 µg to 1000 mg may be administered; in some embodiments, 10, 30, 100 or 1000 µg cna be administered. In some embodiments, 1-5 g of the compositions can be administered. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

The nucleic acid molecules useful in the invention can also include small hairpin RNAs (shRNAs), and expression constructs engineered to express shRNAs. Transcription of shRNAs is initiated at a polymerase III (pol III) promoter, and is thought to be terminated at position 2 of a 4-5-thymine transcription termination site. Upon expression, shRNAs are thought to fold into a stem-loop structure with 3' UU-overhangs; subsequently, the ends of these shRNAs are processed, converting the shRNAs into siRNA-like molecules of about 21 nucleotides. Brummelkamp et al. (2002), Science, 296, 550-553; Lee et al, (2002). *supra*; Miyagishi and Taira (2002), Nature Biotechnol., 20, 497-500; Paddison et al. (2002), *supra*; Paul (2002), *supra*; Sui (2002) *supra*; Yu et al. (2002), *supra*. More information about shRNA design and use may be found the following web sites: katahdin.cshl.org:9331/RNAi/docs/BseRI-BamHI_Strategy.pdf and at katahdin.cshl.org:9331/RNAi/docs/Web_version_of_PCR_strategy1.pdf. Such siRNAs can then be modified as described herein, e.g. by addition of a peptide, or can be mixed with a dendrimer for delivery, e.g., PAMAM, as described herein.

The expression constructs may be any construct suitable for use in the appropriate expression system and include, but are not limited to retroviral vectors, linear expression cassettes, plasmids and viral or virally-derived vectors, as known in the art. Such expression constructs may include one or more inducible promoters, RNA Pol III promoter systems such as U6 snRNA promoters or H1 RNA polymerase III promoters, or other promoters known in the art. The constructs can include one or both strands of the siRNA. Expression constructs expressing both strands can also include loop structures linking both strands, or each strand can be separately transcribed from separate promoters within the same construct. Each strand can also be transcribed from a separate expression construct. (Tuschl (2002), *supra*). Linear constructs may be delivered either by conjugation with a delivery peptide or by mixing with PAMAM; non-linear constructs may be delivered by mixing with PAMAM.

The pharmaceutical compositions can be included in a container, pack, or dispenser together with instructions for administration.

### Methods of Treatment

The present invention is useful for both prophylactic and therapeutic methods of treating a subject at risk of developing (or susceptible to) a disorder, or having a disorder, associated with a dominant gain of function mutation or who would benefit from decreasing expression of a specific nucleic acid sequence or allele of a nucleic acid sequence. As used herein, the term "treatment" is defined as the application or administration of the siRNA compositions to a patient, or application or administration of a therapeutic composition including the siRNA compositions to an isolated tissue or cell line from a patient, who has a disease, a symptom of disease, or a predisposition toward a disease, with the purpose to cure, heal, alleviate, relieve, alter, remedy, ameliorate, improve, or affect the disease, the symptoms of disease, or the predisposition toward disease. The treatment can include administering siRNAs to one or more target sites on one or more target alleles. The mixture of different siRNAs may be administered together or sequentially, and the mixture may be varied for each delivery.

With regards to both prophylactic and therapeutic methods of treatment, such treatments can be specifically tailored or modified, based on knowledge obtained from the field of genomics, particularly genomics technologies such as gene sequencing, statistical genetics, and gene expression analysis, as applied to a patient's genes. Thus, the invention may be used to provide methods for tailoring an individual's prophylactic or therapeutic treatment with the siRNA compositions according to that individual's genotype; e.g., by determining the exact sequence of relevant region of the patient's genome and deigning, using the present methods, an siRNA molecule customized for that patient. This allows a clinician or physician to tailor prophylactic or therapeutic treatments to patients to enhance the effectiveness or efficacy of the present methods.

Also with regards to both prophylactic and therapeutic methods of treatment, such treatments may be specifically tailored or modified, based on knowledge obtained from the field of pharmacogenomics. "Pharmacogenomics," as used herein, refers to the application of genomics technologies such as gene sequencing, statistical genetics, and gene expression analysis to drugs in clinical development and on the market. More specifically, the term refers to the study of how a patient's genes determine his or her response to a drug (e.g., a patient's "drug response phenotype," or "drug response genotype.") Thus, there is provided methods for tailoring an individual's prophylactic or therapeutic treatment with the siRNA compositions according to that individual's drug response genotype. Pharmacogenomics allows a clinician or physician to target prophylactic or therapeutic treatments to patients who will most benefit from the treatment and to avoid treatment of patients who will experience toxic drug-related side effects. For example, if a subject carries two different allele's of a gene, and one allele is associated with undesirable side-effects of a drug to be administered to the subject, expression of the allele can be decreased using the methods described herein during treatment with the drug.

### EXAMPLE

The following materials, methods, and examples are illustrative only and not intended to he limiting.

### Materials and methods for Examples 1-10

### siRNA preparation

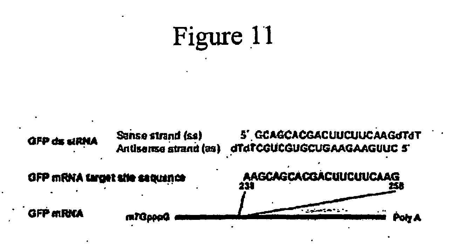

21-nucleotide RNAs were chemically synthesized as 2' bis(acetoxyethoxy)-methyl ether protected oligos by Dharmacon (Lafayette, CO). Synthetic oligonucleotides were deprotected, annealed and purified as described by manufacturer. Successful duplex formation was confined by 20% non-denaturing polyacrylamide gel electrophoresis. All siRNA were stored in diethyl pryocarbonate (DEPC)-treated water at -80C. The sequence of EGFP specific siRNA duplexes was designed following the manufacturer's recommendation and subjected to a BLAST search against the human genome sequence to ensure no gene of the genome was targeted. The siRNA sequence targeting BGFP was from position 238-258 relative to the start cordon as shown in FIG. 11. For RNA interference targeted to endogenous CDK9 [GenBank Accession No. AF255306], the sequence of the CDK9-specific siRNA duplexes was designed following the manufacturer's recommendation and subjected to a BLAST search against the human genome sequence to ensure only CDK9 gene was targeted. The siRNA sequence targeting CDK9 was from position 258-278 relative to the start codon (SEQ ID NO. 15: CCAAAGCUUCCCCCUAUAAdTdT). Duplex siRNAs with 5'Cy3 modification at sense strand were used to determine uptake efficiency while duplex siRNAs with 3'amino modification were used in crosslinking with TAT peptide as described below.

### Crosslinking siRNA with TAT peptide

Modified siRNA containing 3'Amino group with 3-carbon linker (3'N3) were formed by annealing deprotected 3'N3 modified single stranded siRNA with its complementary strand sequence. 25 nmole duplex siRNA with 3'N3 modification were then incubated with 50-fold-molar excess sulfosuccinimidyl 4-[p-maleimidophenyl]butyrate crosslinkers (Sulfo-SMPB, PIERCE) in 400µl PBS reaction buffer (20 mM sodium phosphatate buffer, 0.15 M NaCl, pH7.2). After 1 hour of shaking at room temperature, the reaction mixtures were applied to D-Salt Dextran Desalting column (PIERCE) which were pre-equilibrated in reaction buffer. PBS reaction buffer was applied to the column in 400 µL aliquots to elute the duplex siRNA. The duplex siRNAs were eluted in the fractions 4-6 and are monitored by absorbance at 260. The fraction containing malemide-activated siRNA with crosslinker were pooled and incubated with equal molar ratio of TAT peptide (47-57 amino acid sequence of Tat Protein plus cysteine residue which provides the free sulfhydryl group) at room temperature for 1hour.

### Example 1. siRNA transfection efficiency comparison analysis of PAMAM (Dendrimer) to Lipofectamine

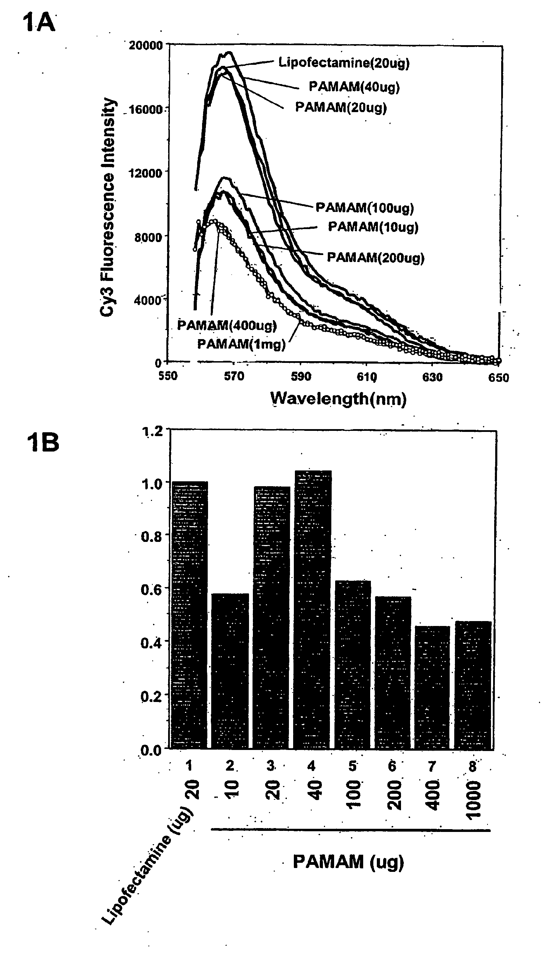

To compare the efficiency of transfection of siRNA in the presence of dendrimers or a standard transfection agent, 21-nucleotide 5'-Cy3-labeled CDK9 sense strand siRNA was deprotected and annealed to unmodified antisense strand and purified as described above. HeLa cells were maintained at 37C in Dulbecco's modified Eagles medium (DMEM, Invitrogen) supplemented with 10% fetal bovine serum (FBS), 100 units/ml penicillin and 100 µg/ml streptomycin (Invitrogen). Cells were regularly passaged at sub-confluence and plated on 60mm plates 16 hr before transfection at 70% confluency. As a control, 20 µg Lipofectamine^{™} (Invitrogen)-mediated transfections of 100 pmole CDK9 5' Cy3-SS/AS duplex siRNAs were performed in 60mm plates as described by the manufacturer for adherent cell lines. For comparison, CDK9 5' Cy3-SS/AS duplex siRNAs were also transfected by mixing with various amount of PAMAM (Sigma-Aldritch) (ranging from 10 µg to 1 mg) using the same conditions as for Lipofectamine^{™} -transfection. Cells were incubated in the transfection mixture for 6 hours and washed three times with PBS (Invitrogen) to remove the transfection mixture. Total nucleotide including DNA, RNA and the transfected siRNAs were isolated by RNA/DNA minikit (QIAGEN) and precipitated by isopropanol. After being dissolved in DEPC-treated water, nucleotide mixtures were subjected to fluorescence measurements on a PTI (Photon Technology International) fluorescence spectrophotometer. The slits were set at 4 nm for both excitation and emission lights. All experiments were carried out at room temperature. Fluorescence of CDK9 5'Cy3-SS/AS duplex siRNA was detected by exciting at 550nm and emission spectrum was recorded from 560nm to 650nm. As shown in FIGS 1A and 1B, the spectrum peak at 570 nm represents the fluorescence intensity of Cy3, which is an indicator of the uptake of CDK9 5'Cy3-SS/AS duplex siRNA as well as the siRNA transfection efficiency by using Lipofectamine^{™} or various amount of PAMAM. The results shown in FIG. 1A indicate the successful transfection of CDK9 5'Cy3-SS/AS duplex siRNA into HeLa cells by PAMAM (dendrimer). The fluorescence of CDK9 5'Cy3-SS/AS duplex siRNA was detected by exciting at 550 nm and emission spectrum was recorded from 560 nm to 650 nm. For control, results from cells subjected to Lipofectamine^{™} -mediated transfection are also shown in black line in FIG. 1A. FIG. 1B is a comparison of siRNA transfection efficiency mediated by PAMAM (dendrimer) to Lipofectamine^{™}. The bars represent the spectrum peak at 570 nm from FIG. 1A (the fluorescence intensity of Cy3, which is an indicator of the uptake of CDK9 5'Cy3-SS/AS duplex siRNA as well as the siRNA transfection efficiency) for each of the listed conditions. After normalizing the Cy3 signal for PAMAM transfection with the Cy3 signal derived from Lipofectamine^{™} -mediated transfection, 20-40 µg PAMAM (dendrimer) (bars 3 and 4) is nearly equal to that of 20 µg Lipofectamine^{™} (bar 1). Using higher amounts of PAMAM may interfere with the siRNA uptake (bars 5-8). These results demonstrate that PAMAM can be used to deliver siRNAs efficiently into living cells.

### Example 2: Silencing of CDK9 Expression by siRNA delivered by PAMAM (Dendrimer)

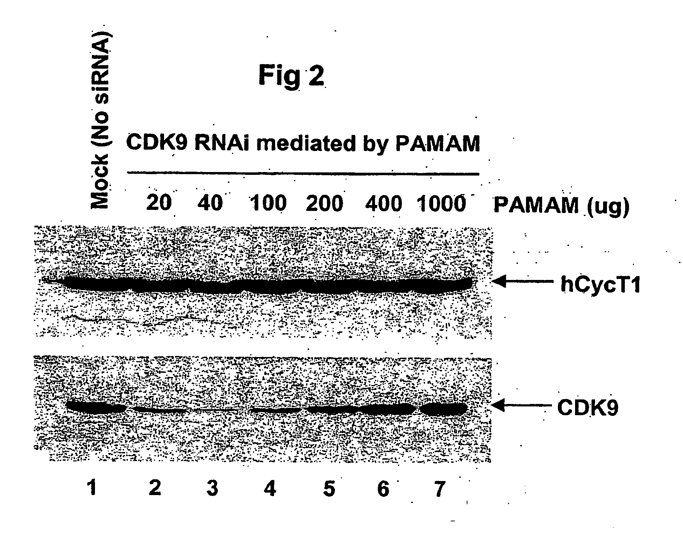

To further investigate the efficacy of using a dendrimer to introduce siRNA into a cell, 100nM CDK9 duplex siRNA were transfected into HeLa cells by Lipofectamine^{™} (as control) or PAMAM (dendrimer) as described above. At 42h post transfection, cells were lysed in ice-cold reporter lysis buffer (Promega) containing protease inhibitor (complete, EDTA-free, 1 tabled/10 mL buffer, Roche Molecular Biochemicals). The resulting lysates were cleared by centrifugation and protein amount in the clear lysate was quantified using a Dc protein assay kit (Bio-Rad). Proteins in 60 µg of total cell lysate were resolved in 10% SDS-PAGE and transferred onto polyvinylidene difluoride membrane (PVDF membrane, Bio-Rad) followed by immunoblotting with antibodies against CDK9 (Santa Cruz). For loading control, the same membrane was also blotted with anti-hCycT1 antibody (Santa Cruz). Protein contents were visualized with BM Chemiluminescence Blotting Kit (Roche Molecular Biochemicals). The blots were exposed to x-ray film (Kodak MR-1) for various times (between 30 seconds and 5 minutes). The results are shown in FIG. 2. For control, cells were treated with 40 µg PAMAM without siRNA (lane 1, mock). These results show the silencing of CDK9 expression by siRNA delivered by PAMAM. This silencing effect was maximal when mediated by 40 µg PAMAM (lane 3) in the reaction mixture. This corresponds to the siRNA uptake efficiency shown in FIG. 1, thus confirming the efficiency of using a dendrimer (e.g., PAMAM) for introducing a nucleic acid such as an siRNA into a cell.

### Example 3: Uptake analysis of siRNAs crosslinked to TAT peptide

To determine whether an siRNA that is cross-linked to a delivery peptide would be taken up by cells, 21-nucleotide 5'Cy3-labeled EGFP sense strand siRNA was deprotected and annealed to 3'N3 modified antisense strand to form duplex siRNAs. The duplex siRNAs were then crosslinked to TAT peptide as described above to form 5'Cy3-SS/AS-TAT siRNA. Hela cells were plated on 60 mm plates 16 hr before transfection at 70% confluency. As control, 20 µg Lipofectamine^{™} (Invitrogen)-mediated transfections of 100 pmole 5' Cy3-SS/AS duplex siRNAs (not conjugated to TAT peptide) were added to cells in 60 mm plates for 6 hr as described above. For comparison, various amounts of 5' Cy3-SS/AS-TAT duplex siRNAs were added in the medium and cells were incubated in the mixture for 6-16 hours and then washed three times with PBS (Invitrogen). Total nucleic acid sequences including DNA, RNA and the transfected siRNAs were isolated by RNA/DNA minikit (Qiagen) and precipitated with isopropanol. After being dissolved in DEPC-treated water, the nucleotide mixtures were subjected to fluorescence measurements on a PTI (Photon Technology International) fluorescence spectrophotometer as described above.

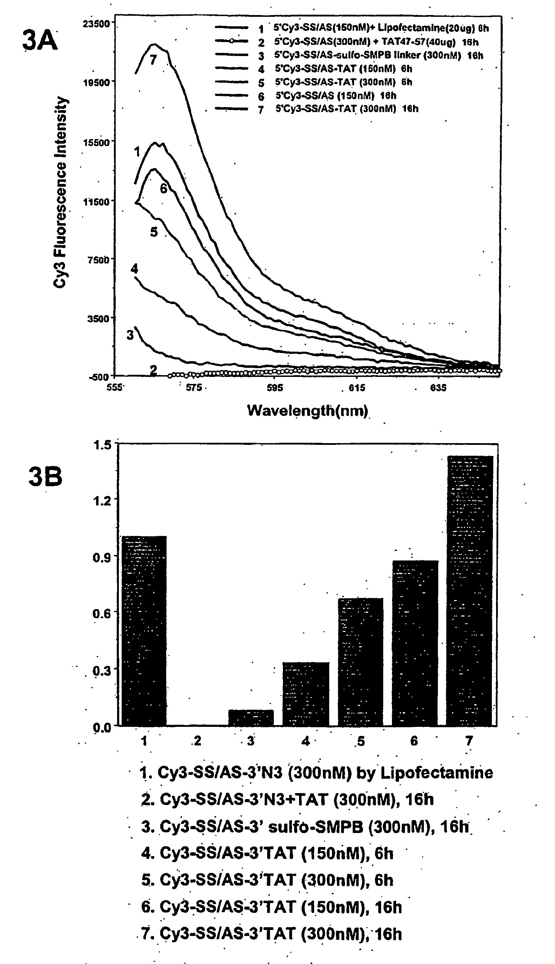

FIG. 3A shows the results of these experiments. Fluorescence intensity of Cy3 indicates the uptake of TAT peptide-crosslinked 5'Cy3-SS/AS duplex siRNA by HeLa cells. Fluorescence of EGFP 5'Cy3-SS/AS duplex siRNA was detected by exciting at 550nm and emission spectrum was recorded from 560nm to 650nm. As a control, 20 µg Lipofectamine^{™} (Invitrogen)-mediated transfections of 100 pmole 5' Cy3-SS/AS duplex siRNAs (without conjugated to TAT peptide) were performed in 60mm plates for 6 hr. Neither mixture of duplex siRNA and TAT peptide (without crosslinking to each other) nor 5'Cy3-SS/AS-Sulfo-SMPB linker showed any uptake into the HeLa cells. FIG. 3B is a comparison of siRNA-TAT uptake efficiency to Lipofectamine^{™}-mediated transfection. The spectrum peak at 570nm from FIG. 1A represents the fluorescence intensity of Cy3, which is an indicator of the uptake of EGFP 5'Cy3-SS/AS-TAT duplex siRNA. The relative efficiency of siRNA uptake can be determined by normalizing the Cy3 signal with the signal derived from Lipofectamine^{™} -mediated transfection. Cells treated with 150 nM siRNA-TAT for 16 hr (bar 6) or with 300 nM siRNA-TAT for 6 hr (bar 5) has almost equal uptake compared to 20 µg Lipofectamine^{™} mediated transfection (bar 1). Using higher concentrations of siRNA-TAT and longer incubation times achieves increased amounts of siRNA uptake (bar 7).

The data demonstrate the conjugation of an siRNA to a delivery peptide is a useful method of introducing an siRNA into a cell.

### Example 4: Determination of RNAi effect of siRNA crosslinked to TAT peptide by dual fluorescence assay

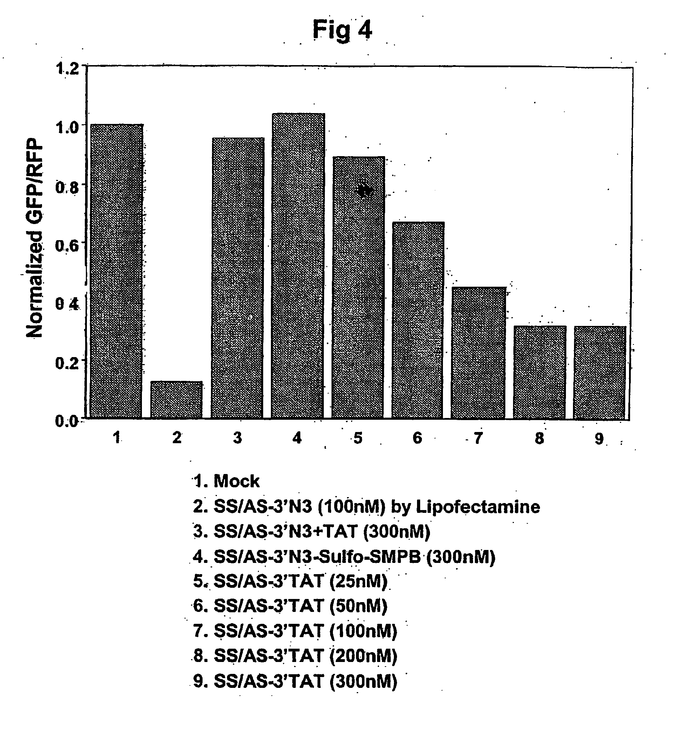

EGFP duplex siRNAs were transfected into HeLa cells by Lipofectamine^{™} (as control) or directly added into the medium after conjugation with TAT peptide and incubated for 16 hrs as described above. At 16h post incubation, pEGFP-C1, pDsRed2-N1 reporter plasmids were cotransfected into HeLa cells. EGFP-C1 encoded enhanced green fluorescence protein (EGFP) while DsRed2-Nl encoded red fluorescence protein (RFP) (Clontech). At 42 hours post transfection, the cells were harvested, the clear lysate was prepared, and then quantified as described above. 300 µg of total cell lysate in 160 µl of reporter lysis buffer were subject to fluorescence measurements on a PTI fluorescence spectrophotometer. The slits were set 4 nm for both excitation and emission lights. All experiments were carried out at room temperature. Fluorescence of EGFP in the cell lysate was detected by exciting at 488 nm and emission spectrum was recorded from 498 nm to 650 nm. The spectrum peak at 507 nm represents the fluorescence intensity of EGFP. Fluorescence of RFP in the same cell lysate was detected be exciting at 568 nm and emission spectrum was recorded from 588 nm-650 nm and the spectrum peak at 583 nm represents the fluorescence intensity of RFP. The fluorescence intensity ratio of target (EGFP) to control (RFP) fluorophore was determined in the presence of siRNA duplex and was normalized to that observed in the absence of siRNA. Normalized ratios less than 1 indicate specific interference. Results are presented in FIG. 4. RNA interference activity was increased by treating the cells with increasing amount of SS/AS-TAT siRNA (bars 5-9). For comparison, RNAi activity of EGFP siRNA transfected by Lipofectamine^{™} was performed (bar 2). Control cells treated with a mixture containing 300 nM EGFP siRNA and TAT peptides (bar 3) or EGFP siRNA conjugated with sulfo-SMPB crosslinker, an intermediate in the crosslinking process, (bar 4), showed no interference activity.

These data demonstrate that siRNAs cross-linked to a delivery peptide can be used to effectively decrease expression of a targeted sequence.

### Example 5: Silencing of CDK9 Expression by siRNA crosslinked to TAT peptide

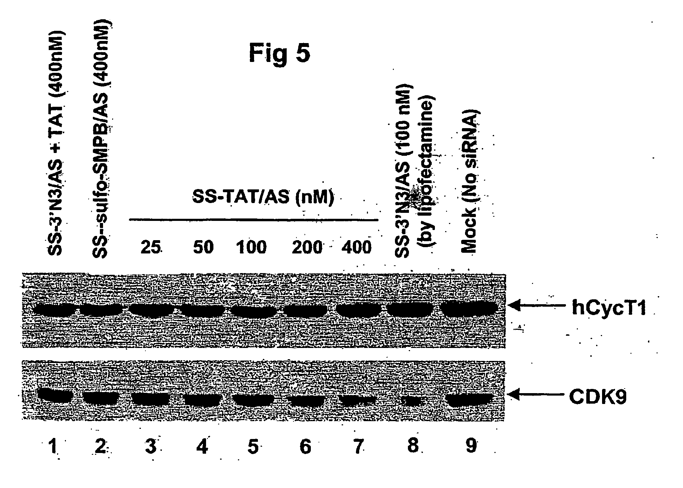

To confirm that siRNAs that are cross-linked to a delivery peptide can be used to decrease expression with a targeted gene, 100nM CDK9 duplex siRNAs were transfected into HeLa cells by Lipofectamine^{™} (as control) or added into the medium without Lipofectamine^{™} after conjugation with TAT peptide. The cells were incubated for 16 hrs as described above. At 42 hours post incubation, proteins in 60 µg of total cell lysate were resolved in 10% SDS-PAGE and transferred onto polyvinylidene difluoride membrane (PVDF membrane, Bio-Rad) followed by immunoblotting with antibodies against CDK9 (Santa Cruz) as described above. The results are shown in FIG. 5. For loading control, the same membrane was also blotted with anti-hCycT1 antibody (Santa Cruz) (Figure 5, upper panel). Protein contents were visualized with BM Chemiluminescence Blotting Kit (Roche Molecular Biochemicals) followed by exposing to x-ray film (Kodak MR-1). RNA interference activity was increased in the cells treated with increasing amount of SS-TAT/AS siRNA. (lanes 3-7). For comparison, RNAi activity of CDK9 SS-3'N3/AS siRNA transfected by Lipofectamine^{™} was performed (lane 8). As a control, cells were treated with a mixture containing 400 nM CDK9 siRNA and free TAT peptides (lane 1) or siRNA conjugated with sulfo-SMPB crosslinker, an intermediate in the crosslinking process. These cells showed no interference activity (lane 2). These data show the silencing of CDK9 expression by siRNA crosslinked with TAT peptide, thus demonstrating that siRNAs cross-linked to delivery peptides are functional in RNAi.

### Example 6: Cellular localization of siRNA in Human cells transfected by lipofectamine.

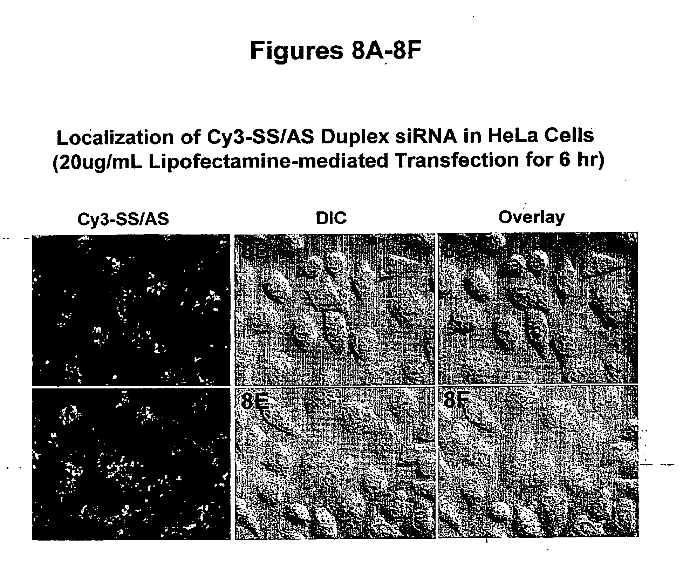

To further investigate the metabolism of siRNAs, 21-nucleotide 5'-Cy3-labeled sense strand siRNA was deprotected and annealed to unmodified antisense strand and applied to HeLa cells at 70% confluency by Lipofectamine^{™} -mediated transfection. The cells were incubated in 1mL of transfection mixture containing 20 µg Lipofectamine^{™} and 100 pmole 5' Cy3-SS/AS duplex siRNAs for 6 hours, and then washed three times with PBS (Invitrogen) to remove the transfection mixture. Cells were fixed in 100% methanol (pre-cooled to -20C) for 10 minutes, air dried and then re-hydrated in PBS. The uptake of siRNA in HeLa cells was monitored by fluorescence microscope by using a Cy3 Filter. Examplary fields are shown in FIG. 8. Cy3 fluorescence represents the localization of duplex siRNA (FIGS. 8a and 8d). Overlay images of the Cy3 signal (Figures 8a and 8d) with the DIC (Figures 8b and 8e) indicated the distribution of siRNA in cytoplasm as well as nuclear region in the cells while transfected by Lipofectamine^{™} (Figures 8c and 8f, pseudocolored).

### Example 7: Dendrimer (PAMAM) mediated siRNA delivery into human cells

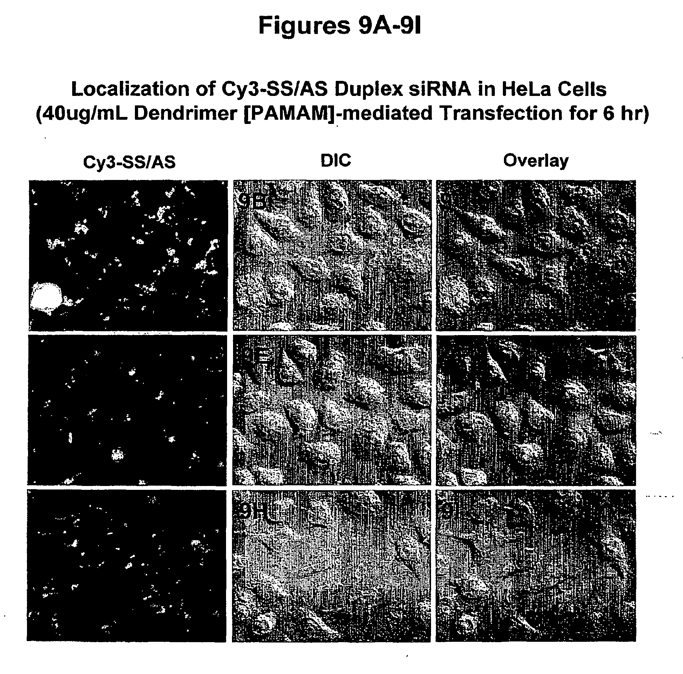

The cellular localization with siRNA introduced into cells using a dendrimer was examed. In these experiments, 21-nucleotide 5'-Cy3-labeled sense strand siRNA was deprotected and annealed to unmodified antisense strand and applied to HeLa cells grown to 70% confluency by PAMAM-mediated transfection as described herein. Cells were incubated in 1mL transfection mixture containing 40 µg PAMAM, 100 pmole 5' Cy3-SS/AS duplex siRNAs for 6 hours at 37°C and washed three times with PBS (Invitrogen) to remove the transfection mixture. Cells were fixed in 100% methanol (pre-cooled to -20C) for 10 minutes, air dried and then re-hydrated in PBS. The uptake of siRNA in HeLa cells was monitored by fluorescence microscope using a Cy3 filter. Exemplary data are shown in FIG. 9. Cy3 Fluorescence represents the localization of duplex siRNA (FIGS. 9A, 9D and 9G). Overlay images of the Cy3 signal (Figures 9a, 9d and 9g) with the DIC (FIGS. 9B, 9E and 9H) indicate that PAMAM-mediated delivery can localize siRNA to both cytoplasm and nuclear regions in the cells (FIGS. 9C, 9F and 9I). Much of the Cy3 fluorescence signal was detected in the nuclear region (FIGS. 9C and 9F) indicating that Lipofectamine^{™} and PAMAM-mediated delivery may have different routings.

### Example 8: TAT (47-57) peptide-mediated siRNA delivery into human cells

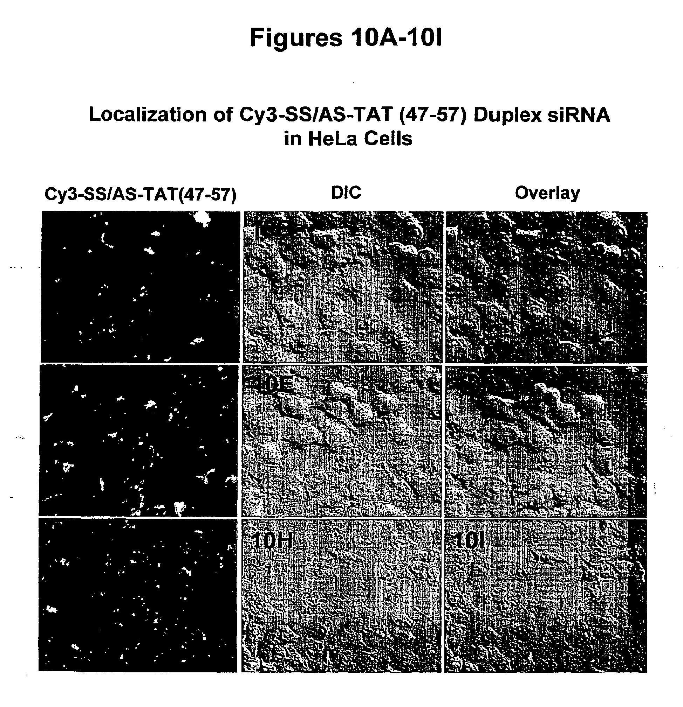

The cellular localization of siRNA introduced into cells using an siRNA cross-linked to a delivery peptide was examined. In these experiments, 21-nucleotide 5'-Cy3-labeled sense strand siRNA was deprotected and annealed to 3' amino modified antisense strand. Duplex siRNAs were crosslinked to TAT(47-57) peptide via sulfosuccinimidyl 4-[p-maleimidophenyl]butyrate crosslinkers (Sulfo-SMPB, PIERCE) as described herein. 100nM 5' Cy3-SS/AS-TAT (47-57) duplex siRNAs were applied to HeLa cells at 70% confluency at 37°C for 6 hours. The cells were washed three times with PBS (Invitrogen) to remove the transfection mixture. Cells were then fixed in 100% methanol (pre-cooled to -20C) for 10 minutes, air dried and then re-hydrated in PBS. The uptake of siRNA in HeLa cells was monitored by fluorescence microscopy using a Cy3 Filter and 400X magnification. As shown in FIG. 10, Cy3 Fluorescence represents the localization of duplex siRNA (FIGS. 10A, 10D and 10G). Overlay images of the Cy3 signal (Figures 10a, 10d and 10g) with the DIC (FIGS. 10G, 10E and 10H) indicate that TAT peptide-mediated delivery can localize siRNA to the cytoplasm (FIGS. 10C, 10F and 10I).

### Example 9: TAT-mediated delivery of siRNA in vivo

To demonstrate the efficiency of delivery of siRNA cross-linked to a delivery peptide, mice are injected intraperitoneally with Cy3-SS/AS-TAT (47-57) duplex siRNA or with control Cy3-SS/AS duplex siRNA in phosphate-buffered saline (PBS). Whole blood cells are isolated from the orbital artery, and splenocytes are isolated at various time points and analyzed by flow cytometry (FACS). Treated mice are sacrificed, tissues are harvested and then frozen in HISTO PREP medium (Fisher Scientific). Sections (10 to 50 mm) are cut on a cryostat, fixed in 0.25% glutaraldehyde for 15 min, and analyzed by fluorescence confocal microscopy. Samples can also be assayed for a decrease in expression of the targeted sequence by comparing the expression (e.g., RNA or protein) in experimental and control animals.

### Example 10: PAMAM-mediated delivery of siRNA in vivo

PAMAM dendrimers have relatively low toxicity and are therefore useful for introducing siRNAs into living systems, including animals. For example, to demonstrate delivery of an siRNA (or other smallnucleic acid) to a mouse, mice are injected intraperitoneally with Cy3-SS/AS duplex siRNA mixed with PAMAM or with control Cy3-SS/AS duplex siRNA in phosphate-buffered saline (PBS). Whole blood cells are isolated from the orbital artery, and splenocytes are isolated at various time points and analyzed by flow cytometry (FACS). Treated mice are sacrificed, tissues are harvested and then frozen in HISTO PREP medium (Fisher Scientific). Sections (10 to 50 mm) are cut on a cryostat, fixed in 0.25% glutaraldehyde for 15 min, and analyzed by fluorescence confocal microscopy to determine whether the siRNA was taken up by cells. In addition, samples can be assayed for a decrease in expression of the targeted sequence by comparing the expression (e.g., RNA or protein) in experimental and control animals.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### ADDITIONAL REFERENCES CITED

1. Lindgren et al., Tips, Volume 21, pp. 99-103, (2000).
2. Schwarze et al., Tips, Volume 21, pp. 45-48, (2000).
3. Prochauntz, Curr Op. Cell Biol., Volume 12, pp. 400-406 (2000).
4. Fawell et al., "TAT-Delivery of Proteins", PNAS USA, Volume 91, pp. 664-668 (1994).
5. Vives et al., J. Biol Chem., Volume 272(25), pp. 1610-1617 (1997).
6. Schwarze et al., Science, Volume 285, pp. 1569-1572 (1999).
7. Nagahara et al., Nature, Volume 4(12), pp. 1449-1452 (1998).
8. Chen et al., PNAS USA, Volume 96, pp. 4325-4329 (1999).
9. Elliott et al., Cell, Volume 88, pp. 223-233 (1997).
10. Morris, Nucl. Acids Res., Volume 25(14), pp. 2730-2736 (1997).
11. Luo, Nature Biotechnology Review, Volume 18, pp. 33-37 (2000).
12. Morris et al., Curr. Op. Biotechnology, Volume 11, pp. 461-466 (2000).
13. Morris et al., Nature Biotechnology, Volume 19, pp. 1173-1176 (2001).
14. Scheller et al., J. Peptide Sci., Volume 5, pp. 185-194 (1999).
15. Oehlke et al., Eur. J. Biochem., Volume 269, pp. 4025-4032 (2002).
16. Tseng et al., Mol. Pharm., Volume 622, pp. 865-872 (2002).
17. Tung et al., Bioorg. And Med. Chem., Volume 10, pp. 3609-3614 (2002).
18. Astriat-Fisher et al., Pharm. Res., Volume 19(6), pp. 744-754 (2002).
19. Park et al., Mol. Cell., Volume 13(2), pp. 202-208, (2002).
20. Liu et al., Biomacromolecules, Volume 2(2), pp. 362-368 (2001).
21. Derossi, J. Biol. Chem., Volume 269(14), pp. 10444-10450 (1994).
22. Vives et al., Nuc. Acids Res., Volume 27(20), pp. 4071-4076 (1999).
23. Sihoder, Eur. J. Biochem., Volume 269, pp. 494-501 (2002).
24. Koppelhus et al., Antisense and Nuc. Acid Drug. Der., Volume 12, pp. 51-63 (2002).
25. Vives et al., Nuc. Acids Res., Volume 27(20), pp. 4071-4076 (1999).
26. Wang et al, J. Am. Chem. Soc., Volume 119, pp. 6444-6445 (1997).
27. Tamilarasu et al., J. Am. Chem. Soc., Volume 121, pp. 1597-1598, (1999).
28. Tamilarasu et al., Bioorg. of Med. Chem. Lett., Volume 11, pp. 505-507 (2001).

## Claims

1. A delivery mixture consisting essentially of:
a dendrimer selected from the group consisting of a generation 2 dendrimer, a generation 3 dendrimer, and a generation 5 dendrimer, wherein the dendrimer is PAMAM, diaminobutane (DAB) or polyethylene glycol (PEG):
an effective amount of a nucleic acid capable of mediating RNA interference (RNAi) wherein the nucleic acid is an RNA molecule selected from the group consisting of a small interfering RNA (siRNA), and short hairpin RNA (shRNA):
and
a pharmaceutically acceptable carrier.

2. The delivery mixture of claim 1, wherein the siRNA comprises a sense strand and an antisense strand, wherein the antisense strand has a sequence sufficiently complementary to a target mRNA sequence to direct target-specific RNAi.

3. The delivery mixture of claim 2, wherein the sense strand and antisense strand are crosslinked.

4. The delivery mixture of claim 3, wherein the siRNA contains a single crosslink.

5. The delivery mixture of claim 3, wherein the sense strand and antisense strand are psoralen crosslinked.

6. The delivery mixture of claim 1, wherein the siRNA comprises a modification at the 3' OH terminus of the sense strand or antisense strand.

7. The delivery mixture of claim 6, wherein the modification at the 3' OH terminus is a biotin, a peptide, a nanoparticle, a peptidomimetic, a dendrimer, a photocleavable biotin, or a dendrimer.

8. The delivery mixture of any one of claims 2 to 7, wherein the siRNA is between about 16 and 30 nucleotides in length, or the siRNA is about 21 nucleotides in length.

9. The delivery mixture of any one of claims 2 to 8, wherein the antisense and sense strands are aligned such that the siRNA has 3' overhangs of between 1 and 4 nucleotides.

10. The delivery mixture of claim 9, wherein the siRNA has 2-nucleotide 3' overhangs.

11. The delivery mixture of claim 10, wherein the 2-nucleotide 3' overhangs are dTdT or UU.

12. The delivery mixture of claim 1, wherein:
the PAMAM and nucleic acid capable of mediating RNA interference are present at a PAMAM: nucleic acid ratio of between about 10 µg and about 1mg PAMAM per 100 pmol nucleic acid;
the PAMAM and nucleic acid capable of mediating RNA interference are present at a PAMAM: nucleic acid ratio of between about 20 µg and about 40 µg PAMAM per 100 pmol nucleic acid; or
the PAMAM and nucleic acid capable of mediating RNA interference are present at a PAMAM: nucleic acid ratio of about 40 µg PAMAM per 100 pmol nucleic acid.

13. The delivery mixture according to any one of claims 1 to 12 for use in therapy.

14. A pharmaceutical composition comprising the delivery mixture of any one of claims 1-12.

15. An *in vitro* method for delivering a nucleic acid capable of mediating RNA interference (RNAi) to a cell, the method comprising:
(a) obtaining a cell;
(b) forming a delivery mixture according to any of claims 1 to 12; and
(c) contacting the cell with the delivery mixture, thereby delivering the nucleic acid capable of mediating RNAi to the cell.

## Patentansprüche

1. Ein Verabreichungsgemisch, bestehend im Wesentlichen aus:
einem Dendrimer, das aus der Gruppe ausgewählt ist, bestehend aus einem Dendrimer der zweiten Generation, einem Dendrimer der dritten Generation und einem Dendrimer der fünften Generation, wobei das Dendrimer PAMAM, Diaminobutan (DAB) oder Polyethylenglycol (PEG) ist;
einer wirksamen Menge einer Nukleinsäure, die in der Lage ist, eine RNA-Interferenz (RNAi) zu vermitteln, wobei die Nukleinsäure ein RNA-Molekül ist, das aus der Gruppe ausgewählt ist, bestehend aus einer kleinen interferierenden RNA (siRNA) und einer kurzen Haarnadel-RNA (shRNA); und
einem pharmazeutisch verträglichen Träger.

2. Das Verabreichungsgemisch nach Anspruch 1, wobei die siRNA einen Sense-Strang und einen Antisense-Strang umfasst, wobei der Antisense-Strang eine Sequenz besitzt, die zu einer Ziel-mRNA-Sequenz hinreichend komplementär ist, um eine zielspezifische RNAi zu steuern.

3. Das Verabreichungsgemisch nach Anspruch 2, wobei der Sense-Strang und Antisense-Strang vernetzt sind.

4. Das Verabreichungsgemisch nach Anspruch 3, wobei die siRNA eine einzige Vernetzungsstelle enthält.

5. Das Verabreichungsgemisch nach Anspruch 3, wobei der Sense-Strang und Antisense-Strang Psoralen-vernetzt sind.

6. Das Verabreichungsgemisch nach Anspruch 1, wobei die siRNA eine Modifikation am 3'-OH-Terminus des Sense-Stranges oder Antisense-Stranges umfasst.

7. Das Verabreichungsgemisch nach Anspruch 6, wobei die Modifikation am 3'-OH-Terminus ein Biotin, ein Peptid, ein Nanopartikel, ein Peptidomimetikum, ein Dendrimer, ein photochemisch spaltbares Biotin oder ein Dendrimer ist.

8. Das Verabreichungsgemisch nach einem der Ansprüche 2 bis 7, wobei die siRNA eine Länge zwischen etwa 16 und 30 Nukleotiden aufweist oder die siRNA eine Länge von etwa 21 Nukleotiden aufweist.

9. Das Verabreichungsgemisch nach einem der Ansprüche 2 bis 8, wobei der Antisense- und Sense-Strang aliniert sind, sodass die siRNA 3'-Überhänge von zwischen 1 und 4 Nukleotiden besitzt.

10. Das Verabreichungsgemisch nach Anspruch 9, wobei die siRNA 2-Nukleotid-3'- Überhänge besitzt.

11. Das Verabreichungsgemisch nach Anspruch 10, wobei die 2-Nukleotid-3'-Überhänge dTdT oder UU sind.

12. Das Verabreichungsgemisch nach Anspruch 1, wobei:
das PAMAM und die Nukleinsäure, die in der Lage ist, eine RNA-Interferenz zu vermitteln, in einem PAMAM/Nukleinsäure-Verhältnis zwischen etwa 10 µg und etwa 1 mg PAMAM pro 100 pmol Nukleinsäure vorliegen;
das PAMAM und die Nukleinsäure, die in der Lage ist, eine RNA-Interferenz zu vermitteln, in einem PAMAM/Nukleinsäure-Verhältnis zwischen etwa 20 µg und etwa 40 µg PAMAM pro 100 pmol Nukleinsäure vorliegen; oder
das PAMAM und die Nukleinsäure, die in der Lage ist, eine RNA-Interferenz zu vermitteln, in einem PAMAM/Nukleinsäure-Verhältnis von etwa 40 µg PAMAM pro 100 pmol Nukleinsäure vorliegen.

13. Das Verabreichungsgemisch gemäß einem der Ansprüche 1 bis 12 zur therapeutischen Verwendung.

14. Eine pharmazeutische Zusammensetzung, umfassend das Verabreichungsgemisch nach einem der Ansprüche 1 bis 12.

15. Ein *in vitro* Verfahren zur Verabreichung einer Nukleinsäure, die in der Lage ist, eine RNA-Interferenz (RNAi) zu vermitteln, an eine Zelle, wobei das Verfahren umfasst:
(a) Gewinnen einer Zelle;
(b) Herstellen eines Verabreichungsgemisches gemäß einem der Ansprüche 1 bis 12; und
(c) Inkontaktbringen der Zelle mit dem Verabreichungsgemisch, wobei **dadurch** der Zelle die Nukleinsäure verabreicht wird, die in der Lage ist, eine RNAi zu vermitteln.

## Revendications

1. Mélange à administrer consistant essentiellement en :
un dendrimère choisi dans le groupe comprenant un dendrimère de deuxième génération, un dendrimère de troisième génération, et un dendrimère de cinquième génération, le dendrimère étant une PAMAM, un diaminobutane (DAB) ou un polyéthylène glycol (PEG) ;
une quantité efficace d'un acide nucléique capable d'induire une interférence par l'ARN (ARNi) dans laquelle l'acide nucléique est une molécule d'ARN choisie dans le groupe comprenant un ARN court interférent (ARNsi), et un ARN court en épingle à cheveux (ARNsh) ; et
un véhicule pharmaceutiquement acceptable.

2. Mélange à administrer selon la revendication 1, dans lequel l'ARNsi comprend un brin sens et un brin antisens, le brin antisens ayant une séquence suffisamment complémentaire d'une séquence d'ARNm cible pour obtenir un ARNi spécifique de la cible.

3. Mélange à administrer selon la revendication 2, dans lequel le brin sens et le brin antisens sont réticulés.

4. Mélange à administrer selon la revendication 3, dans lequel l'ARNsi contient une réticulation simple.

5. Mélange à administrer selon la revendication 3, dans lequel le brin sens et le brin antisens sont réticulés au psoralène.

6. Mélange à administrer selon la revendication 1, dans lequel l'ARNsi comprend une modification à l'extrémité 3' OH du brin sens ou du brin antisens.

7. Mélange à administrer selon la revendication 6, dans lequel la modification à l'extrémité 3' OH est une biotine, un peptide, une nanoparticule, un composé peptidomimétique, un dendrimère, une biotine pouvant être coupée de manière photochimique, ou un dendrimère.

8. Mélange à administrer selon l'une quelconque des revendications 2 à 7, dans lequel l'ARNsi représente entre environ 16 et 30 nucléotides de longueur, ou l'ARNsi représente environ 21 nucléotides de longueur.

9. Mélange à administrer selon l'une quelconque des revendications 2 à 8, dans lequel les brins antisens et sens sont alignés de sorte que l'ARNsi présente des saillies 3' représentant 1 à 4 nucléotides.

10. Mélange à administrer selon la revendication 9, dans lequel l'ARNsi présente des saillies 3' de 2 nucléotides.

11. Mélange à administrer selon la revendication 10, dans lequel les saillies 3' de 2 nucléotides sont dTdT ou UU.

12. Mélange à administrer selon la revendication 1, dans lequel :
la PAMAM et un acide nucléique capable d'induire une interférence par l'ARN sont présents dans un ratio PAMAM : acide nucléique entre environ 10 µg et environ 1 mg de PAMAM pour 100 pmol d'acide nucléique ;
la PAMAM et un acide nucléique capable d'induire une interférence par l'ARN sont présents dans un ratio PAMAM : acide nucléique entre environ 20 µg et environ 40 µg de PAMAM pour 100 pmol d'acide nucléique ; ou
la PAMAM et un acide nucléique capable d'induire une interférence par l'ARN sont présents dans un ratio PAMAM : acide nucléique d'environ 40 µg de PAMAM pour 100 pmol d'acide nucléique.

13. Mélange à administrer selon l'une quelconque des revendications 1 à 12 destiné à être utilisé en thérapie.

14. Composition pharmaceutique comprenant le mélange à administrer selon l'une quelconque des revendications 1 à 12.

15. Procédé *in vitro* destiné à administrer un acide nucléique capable d'induire une interférence par l'ARN (ARNi) dans une cellule, le procédé comprenant les étapes suivantes :
(a) obtenir une cellule ;
(b) former un mélange à administrer selon l'une quelconque des revendications 1 à 12 ; et
(c) mettre en contact la cellule avec le mélange à administrer, administrant de ce fait l'acide nucléique capable d'induire un ARNi dans la cellule.


### SEQUENCE LISTING

<110> University of Massachusetts, Inc.
<120> DELIVERY OF siRNAs
<130> UMY-059PC
<150> 60/430520
   <151> 2002-11-26
<160> 16
<170> FastSEQ for Windows Version 4.0
<210> 1
   <211> 13
   <212> PRT
   <213> Artificial Sequence
<220>
   <223> peptide
<400> 1
   Arg Lys Lys Arg Arg Gln Arg Arg Arg Pro Pro Gln Cys
    1               5                  10
<210> 2
   <211> 19
   <212> PRT
   <213> Artificial Sequence
<220>
   <223> peptide
<400> 2
   Arg Gln Ile Lys Ile Trp Phe Gln Asn Arg Arg Met Lys Trp Lys Lys
    1               5                  10                  15
   Gly Gly Cys
<210> 3
   <211> 34
   <212> PRT
   <213> Artificial Sequence
<220>
   <223> peptide
<400> 3
   Asp Ala Ala Thr Ala Thr Arg Gly Arg Ser Ala Ala Ser Arg Pro Thr
   1 5 10 15
   Glu Arg Pro Arg Ala Pro Ala Arg Ser Ala Ser Arg Pro Arg Arg Pro
   20 25 30
   Val Glu
<210> 4
   <211> 21
   <212> PRT
   <213> Artificial Sequence
<220>
   <223> peptide
<400> 4
   Lys Glu Thr Trp Trp Glu Thr Trp Trp Thr Glu Trp Ser Gln Pro Lys
   1 5 10 15
   Lys Lys Arg Lys Val
   20
<210> 5
   <211> 27
   <212> PRT
   <213> Artificial Sequence
<220>
   <223> peptide
<400> 5
   Gly Ala Leu Phe Leu Gly Trp Leu Gly Ala Ala Gly Ser Thr Met Gly
   1 5 10 15
   Ala Trp Ser Gln Pro Lys Lys Lys Arg Lys Val
   20 25
<210> 6
   <211> 16
   <212> PRT
   <213> Artificial Sequence
<220>
   <223> peptide
<400> 6
   Ala Ala Val Ala Leu Leu Pro Ala Val Leu Leu Ala Leu Leu Ala Pro
   1 5 10 15
<210> 7
   <211> 16
   <212> PRT
   <213> Artificial Sequence
<220>
   <223> peptide
<400> 7
   Arg Gln Ile Lys Ile Trp Phe Gln Asn Arg Arg Met Lys Trp Lys Lys
   1 5 10 15
<210> 8
   <211> 27
   <212> PRT
   <213> Artificial Sequence
<220>
   <223> peptide
<400> 8
   Gly Ala Leu Phe Leu Gly Trp Leu Gly Ala Ala Gly Ser Thr Met Gly
   1 5 10 15
   Ala Trp Ser Gln Pro Lys Lys Lys Arg Lys Val
   20 25
<210> 9
   <211> 16
   <212> PRT
   <213> Artificial Sequence
<220>
   <223> peptide
<400> 9
   Ala Ala Val Ala Leu Leu Pro Ala Val Leu Leu Ala Leu Leu Ala Pro
   1 5 10 15
<210> 10
   <211> 26
   <212> PRT
   <213> Artificial Sequence
<220>
   <223> peptide
<400> 10
   Gly Trp Thr Leu Asn Ser Ala Gly Tyr Leu Leu Lys Ile Asn Leu Lys
   1 5 10 15
   Ala Leu Ala Ala Leu Ala Lys Lys Ile Leu
   20 25
<210> 11
   <211> 18
   <212> PRT
   <213> Artificial Sequence
<220>
   <223> peptide
<400> 11
   Lys Leu Ala Leu Lys Leu Ala Leu Lys Ala Leu Lys Ala Ala Leu Lys
   1               5                   10                  15
   Leu Ala
<210> 12
   <211> 12
   <212> PRT
   <213> Artificial Sequence
<220>
   <223> peptide
<400> 12
   Cys Tyr Gly Arg Lys Lys Arg Arg Gln Arg Arg Arg
   1               5                   10
<210> 13
   <211> 21
   <212> RNA
   <213> Artificial Sequence
<220>
   <223> siRNA
<221> misc_feature
   <222> 20, 21
   <223> n = deoxythymidine
<400> 13
   gcagcacgac uucuucaagn n 21
<210> 14
   <211> 21
   <212> RNA
   <213> Artificial Sequence
<220>
   <223> siRNA
<221> misc feature
   <222> 20, 21
   <223> n =deoxythymidine
<400> 14
   cuugaagaag ucgugcugcn n 21
<210> 15
   <211> 21
   <212> RNA
   <213> Artificial Sequence
<220>
   <223> siRNA
<221> misc_feature
   <222> 20, 21
   <223> n = deoxythymidine
<400> 15
   ccaaagcuuc ccccuauaan n 21
<210> 16
   <211> 12
   <212> PRT
   <213> Artificial Sequence
<220>
   <223> peptide
<400> 16
   Cys Tyr Gln Arg Lys Lys Arg Arg Gln Arg Arg Arg
    1               5                   10